# EUROPÄISCHE PATENTSCHRIFT

(11) **EP 0 721 785 B1**
(45) Veröffentlichungstag und Bekanntmachung des Hinweises auf die Patenterteilung: **27.03.2002**
(21) Anmeldenummer: 95119604.7
(22) Anmeldetag: 13.12.1995
(51) Int. Cl.: A61K 47/48

(54) **Entbittertes Ranitidin-Präparat**
Debittered ranitidine preparation
Préparation de ranitidin désamerisée

(30) Priorität: 17.12.1994 DE 9420259 U
(43) Veröffentlichungstag der Anmeldung: 17.07.1996
(73) Patentinhaber: Röhm GmbH & Co. KG, 64293 Darmstadt (DE)
(72) Erfinder: Assmus, Manfred, D-64404 Bickenbach (DE); Petereit, Hans-Ulrich, D-64291 Darmstadt (DE)

(56) Entgegenhaltungen:
- EP-A- 0 193 400
- WO-A-93/24124
- CH-A- 664 284
- GB-A- 2 207 865
- US-A- 4 539 199

## Beschreibung

Die Erfindung betrifft ein von Bittergeschmack weitgehend oder vollständig freies Ranitidin-Präparat, insbesondere in Form einer wäßrigen Lösung. Ranitidin ist der Substanzname (generic name) von N-[2-(5-Dimethylaminomethylfurfurylthio)-ethyl]-N'-methyl-2-nitro-1,1-ethylendiamin, das zur Behandlung von Gastritis dient. Die bevorzugte Einsatzform ist Ranitidin-hydrochlorid, das allerdings als wäßrige Lösung extrem bitter schmeckt und deshalb in dieser Form kaum zur Anwendung kommt. Das Hydrochlorid wird vorwiegend in umhüllter oder mikrogekapselter Form eingenommen, beispielsweise in Form von Filmtabletten oder überzogenen Pellets.

### Stand der Technik

Zur Herstellung geschmacksneutraler Präparate aus basischen Wirkstoffen mit ausgeprägtem Bittergeschmack ist es aus CH-A 664 284, DE-A 27 52 705 und US-A 4,539,199 bekannt, aus wäßrigen Lösungen der Wirkstoffe mittels Polyanionen, insbesondere neutralisierten Copolymerisaten der Acryl- und/oder Methacrylsäure mit niederen Alkylestern dieser Säuren, unlösliche Salze auszufällen, die zu Tabletten oder Suspensionen verarbeitet werden. In dieser Form sind die unlöslichen Salze geschmacklos, werden jedoch nach der Einnahme im Magen wieder in ihre ursprünglichen Bestandteile aufgespalten, so daß der Wirkstoff resorbiert werden kann. In dieser Weise wurden Präparate von Chloroquine, Ephedrin, Doxepin, Chloropromazin, Trimipramin, Quinidin, Benzyclan, Papaverin, Chloranolol und anderen Wirkstoffen hergestellt. Schwer- oder unlösliche Salze dieser Art werden gemäß EP-A 417588 durch Umsetzung in einer angefeuchteten Pulvermischung hergestellt. Die Verarbeitung der unlöslichen Salze zu Suspensionen ist wegen der Gefahr der allmählichen Entmischung nicht immer befriedigend.

Aus US-A 3,629,392 ist ein Verfahren zur Herstellung von Retard-Präparaten bekannt, bei dem ein basischer Wirkstoff mit einer wäßrigen Dispersion eines saure Gruppen enthaltenden Polymeren umgesetzt wird. Dabei wird der Wirkstoff an die Oberfläche der dispergierten Latexteilchen gebunden. In vielen Fällen koaguliert die Dispersion von selbst. Andernfalls werden Koagulationsmittel zugesetzt, um ein festes Umsetzungsprodukt zu gewinnen. Die Verabreichung eines unkoagulierten wirkstoffhaltigen Latex als Flüssigpräparat wird nicht beschrieben.

WO 93/24124 beschreibt orale Arzneiformen, die aus einem Wirkstoff/lonenaustauscher-Komplex bestehen. Dabei kann es sich um Ranitidin-Salz in Kombination mit bevorzugt vernetzten lonenaustauschharzen handeln. Die genannten handelsüblichen Polyacrylsäuren weisen Molekulargewichte im Bereich mehrerer Millionen auf.

EP 0 193 400 A2 beschreibt orale Arzneiformen, die aus hochmolekularen Ranitidin/Na-Polyacrylsäure-Komplexen bestehen können. Es werden Molekulargewichte im Bereich von 3.000.000 bis 8.000.000 genannt.

### Aufgabe und Lösung

Ziel der Erfindung ist die Bereitstellung eines Ranitidin-Präparates, das in Form einer wäßrigen Lösung weitgehend oder vollständig frei von Bittergeschmack ist. Es wurde nun überraschenderweise gefunden, daß - im Gegensatz zu zahlreichen anderen basischen Wirkstoffen - die Salze des Ranitidins mit Polycarbonsäuren mit einem Molekulargewicht (Gewichtsmittel) von mindestens 1.000 bis 200.000 Dalton wasserlöslich und nach der Einnahme resorbierbar sind und somit die gestellte Forderung erfüllen.

Für Ranitidin-hydrochlorid wird nach Pharm.Eur. ein Bitterwert von 100 000 festgestellt. Dagegen weisen die erfindungsgemäßen Ranitidin-Präparate Bitterwerte unter 1000 auf, was als gut einnehmbar gelten kann.

Der Bittergeschmack des Ranitidin-hydrochlorids kann nur auf die Geschmackswirkung des Ranitidin-Kations zurückgeführt werden, da das Chloridanion bekanntermaßen nicht bitter sondern salzig schmeckt. Es war deshalb überraschend, daß der Bittergeschmack durch Austausch des Anions unterdrückt werden kann, während das Ranitidin-Kation unverändert bleibt. Es wird vermutet, daß das Polyanion der Polycarbonsäure wegen seiner Molekülgröße nicht mit den Geschmacksrezeptoren in Wechselwirkung treten kann und daß die Ranitidin-Kationen in der wäßrigen Lösung so stark an das Polyanion gebunden bleiben, daß sie nicht allein die Bittergeschmack-Rezeptoren erreichen können. Die Erfindung ist allerdings nicht an die Richtigkeit dieser Deutung gebunden. Beim Einbringen in künstlichen Magensaft (0,1 n HCl) läßt sich der freie Wirkstoff nach wenigen Minuten nahezu vollständig nachweisen.

Durch die Erfindung ist es möglich geworden, flüssige, homogene Ranitidin-Präparate zur oralen Applikation in Form von Säften oder Sirupen herzustellen. Weiterhin können aus dem getrockneten Salz Tabletten oder andere feste Arzneiformen zubereitet werden, die auch ohne Umhüllung oder Verkapselung nicht bitter schmecken.

### Ausführung der Erfindung

Die Salze können hergestellt werden, indem man Ranitidin-hydrochorid oder eine andere wasserlösliche Form des Wirkstoffes oder die freie Wirkstoffbase mit einer Lösung, Suspension oder Dispersion der Polycarbonsäure umsetzt. Für eine vollständige Bindung ist pro Mol Ranitidin wenigstens die äquivalente Menge an Carboxylatgruppen der Polycarbonsäure erforderlich. Vorzugsweise wird ein Überschuß der Polycarbonsäure eingesetzt, beispielsweise 2 bis 5 Carboxylat-Äquivalente pro Mol Ranitidin. Die Umsetzung läuft in wäßriger Lösung sehr schnell ab und kann am Nachlassen des Bittergeschmackes verfolgt werden.

Als Polycarbonsäure kommen solche in Betracht, die wenigstens in neutralisierter Form im schwach alkalischen Bereich wasserlöslich und physiologisch verträglich sind. Die Polycarbonsäuren weisen ein Molekulargewicht von mindestens 1000 Dalton bis 200.000 Dalton und bevorzugt ein Carboxyl-Äquivalentgewicht von 72 bis 800, vorzugsweise 100 bis 500 Dalton auf. Die Molekulargewichte (als Gewichtsmittelwerte) liegen z.B. bei 10.000 bis 200.000 Dalton, vorzugsweise bei 50.000 bis 150.000 Dalton. Bei Molekulargewichten unter 1000 läßt die entbitternde Wirkung nach. Polycarbonsäuren mit einem Molekulargewicht über 200 000 ergeben sehr hochviskose Lösungen, die schwer zu handhaben sind.

Bevorzugt sind alkalilösliche Polymerisate oder Copolymerisate der Acryl- und/oder Methacrylsäure, insbesondere aus 10 bis 100, vorzugsweise 30 bis 70 Gew.-% Einheiten der Acryl- und/oder Methacrylsäure und 90 bis 0, vorzugsweise 70 bis 30 Gew.-% Einheiten eines oder mehrerer niederer Alkylester der Acryl- und/oder Methacrylsäure. Bevorzugt sind C₁- bis C₄-Alkylester, insbesondere Methyl- und Ethylester. Unvernetzte oder allenfalls schwach vernetzte, wenigstens kolloidal lösliche Polyacrylsäure und Polymethacrylsäure sind als Handelsprodukte erhältlich. Geeignete Copolymerisate sind als Handelsprodukte der Röhm GmbH, Darmstadt, unter den Warenzeichen EUDRAGIT S bzw. -L und EUDISPERT hv erhältlich und für pharmazeutische Präparate zugelassen. Beispiele derartiger Copolymerisate sind:
Poly-(methylmethacrylat-methacrylsäure) 70:30, EUDRAGIT S100
Poly-(methylmethacrylat-methacrylsäure) 50:50, EUDRAGIT L100
Poly-(methylmethacrylat-methacrylsäure) 30:70, EUDISPERT hv
Poly-(ethylacrylat-methacrylsäure) 50:50, EUDRAGIT L100-55
Poly-(ethylacrylat-methacrylsäure) 50:50, EUDRAGIT L30D

Wenn die Polycarbonsäure in Form eines Pulvers oder einer wäßrigen Dispersion mit der Ranitidin-hydrochlorid-Lösung umgesetzt wird, läßt sich unter Rühren und Erwärmen auf 40 bis 80°C, vorzugsweise 50 bis 90°C und Zusatz einer Base eine klare, farblose oder höchstens schwach gelbliche homogene Lösung des Ranitidin-Salzes erhalten. Die manchmal auftretende Schaumbildung kann durch Rühren bei Unterdruck vermindert werden. Die Menge der Base wird vorzugsweise so begrenzt, daß gerade eine klare Lösung erreicht wird; in der Regel sind 2 Basenäquivalente je Mol des eingesetzten Ranitidin-Salzes ausreichend. Als Base kommen vorzugsweise Alkalihydroxyde oder -carbonate in Betracht. Wird die freie Ranitidin-Base verarbeitet, ist der Alkalizusatz im allgemeinen entbehrlich. Der pH-Wert der Lösung beträgt in der Regel 4 bis 8. Der Feststoffgehalt der Lösung kann im Bereich von 1 bis 60, vorzugsweise 5 bis 30 Gew.-% liegen.

Die Lösung kann als solche zum Einnehmen verwendet werden. Häufig werden zur Verbesserung der Akzeptanz weitere Zusätze zugefügt, z.B. Zucker, wie Saccharose oder Glukose, Süßstoffe, wie Saccharin oder Na-Cyclamat, Geschmackstoffe, Aromen, Farbstoffe, Stabilisatoren, Verdickungsmittel u.ä. Als Handelsform eignen sich Flaschen, gegebenenfalls mit Dosiereinrichtung, oder Ampullen mit Einzeldosen. Die Lösung kann jedoch auch - beispielsweise durch Gefriertrocknung - schonend zu einem Festprodukt eingetrocknet und zu einem Pulver gemahlen werden. Daraus können unter Zusatz üblicher Hilfsstoffe Tabletten gepreßt werden, die ohne Bittergeschmack eingenommen oder gegebenenfalls gekaut oder nach Zerfallen in Wasser eingenommen werden können.

### BEISPIELE

1. Herstellung der Lösung:
   In einem heizbaren und evakuierbaren Kessel werden 448 g Ranitidin HCl in 1100 g gereinigtem Wasser gelöst und unter Rühren auf 65 Grad C erhitzt. Danach werden 237 g EUDRAGIT L 100® eingerührt und weitere 10 Minuten gerührt. Dann erfolgt unter reduziertem Druck die Zugabe einer Lösung von 67,6 g Natriumkarbonat in 147,4 g Wasser. Diese Suspension wird 1 Stunde bei 70 Grad C gerührt.
      Die erhaltene klare Lösung ist farblos bis leicht gelblich gefärbt und hat einen Bitterwert von ca. 500.
2. 5,0 g Ranitidin HCl, 4,4 g EUDRAGIT S 100 und 0,755 g Natriumkarbonat werden analog in 225 g gemischtem Wasser umgesetzt Anschließend löst man in der erhaltenen klaren Flüssigkeit 260,0 g Saccharose. Der endgültige Saft hat einen angenehm süßen und leicht bitteren Geschmack.
3. 5,0 g Ranitidin HCl werden in einem heizbaren und evakuierbaren Kessel in 225 g gereinigtem Wasser gelöst und unter Rühren auf 65 Grad C erhitzt. Danach werden 8,84 g EUDRAGIT L 30® D-55 hinzugefügt und weitere 10 Minuten gerührt. Dann erfolgt unter reduziertem Druck die Zugabe von 0,755 Natriumkarbonat. Diese Suspension wird 1 Stunde bei 70 Grad C gerührt.
   Die erhaltene klare Lösung ist farblos bis leicht gelblich gefärbt und hat nur einen leicht bitteren Geschmack.
4. 5,0 g Ranitidin HCl, 1,99 g EUDISPERT® hv und 0,755 g Natriumkarbonat werden analog zu 2. umgesetzt. Anschließend löst man in der erhaltenen klaren Flüssigkeit 260,0 g Saccharose. Der endgültige Saft hat einen angenehm süßen und leicht bitteren Geschmack.
5. Herstellung eines Sirups:
   900 g einer nach 1. hergestellten Lösung werden auf 50 Grad C erwärmt, mit 900 g Saccharose Pharm. Eur. versetzt und bis zu klaren Lösung (ca. 10 Minuten) gerührt. Die erhaltene Lösung mit leicht erhöhter Viskosität hat einen süßen, leicht bitteren Geschmack. Der Wirkstoffgehalt liegt bei ca. 10 %. Die Freigabe, photometrisch gemessen in künstlichem Darmsaft (BP 88) erfolgt zu 100 % nach 5 Minuten.
6. Herstellung von Kautabletten
   Eine Lösung nach 1. wird gefriergetrocknet und der erhaltene Feststoff fein gemahlen. Zu 267 g dieses Pulvers gibt man 52,3 g Sorbitol Granulat und eine Vormischung (30 Minuten) aus 20 g Maisstärke und 1 g Na-Cyclamat. Die Pulver werden in einem Doppelkonusmischer über 60 Minuten und durch ein 1,5 mm Sieb gegeben. Anschließend gibt man 1 g Magnesiumstearat hinzu und mischt 10 Minuten. Dieses Pulver wird anschließend zu Tabletten verpreßt.

## Patentansprüche

1. Ranitidin-Präparat, enthaltend das Salz des Ranitidins mit einer Polycarbonsäure mit einem Molekulargewicht (Gewichtsmittel) von mindestens 1.000 bis 200.000 Dalton.

2. Ranitidin-Präparat nach Anspruch 1 in Form einer wäßrigen Lösung.

3. Ranitidin-Präparat nach Anspruch 1 oder 2, **dadurch gekennzeichnet, daß** es pro Mol Ranitidin wenigstens die äquivalente Menge an Carboxylatgruppen der Polycarbonsäure enthält.

4. Ranitidin-Präparat nach Anspruch 3, **dadurch gekennzeichnet, daß** die Polycarbonsäure ein Carboxyl-Äquivalentgewicht von 72 bis 800 Dalton hat.

5. Ranitidin-Präparat nach einem oder mehreren der Ansprüche 1 bis 4, **dadurch gekennzeichnet, daß** die Polycarbonsäure ein alkalilösliches Polymerisat oder Copolymerisat der Acryl- und/oder Methacrylsäure ist.

6. Ranitidin-Präparat nach Anspruch 5, **dadurch gekennzeichnet, daß** die Polycarbonsäure ein Polymerisat oder Copolymerisat aus 10 bis 100 Gew.-% Einheiten der Acryl- und/oder Methacrylsäure und 90 bis 0 Gew.-% Einheiten eines oder mehrerer niederer Alkylester der Acryl- und/oder Methacrylsäure ist.

## Claims

1. Ranitidine preparation containing the salt of ranitidine with a polycarboxylic acid having a molecular weight (weight average) of at least 1,000 to 200,000 Daltons.

2. Ranitidine preparation according to claim 1 in the form of an aqueous solution.

3. Ranitidine preparation according to claim 1 or 2, **characterised in that** per mol of ranitidine it contains at least the equivalent amount of carboxylate groups of polycarboxylic acid.

4. Ranitidine preparation according to claim 3, **characterised in that** the polycarboxylic acid has a carboxyl equivalent weight of 72 to 800 Daltons.

5. Ranitidine preparation according to one or more of claims 1 to 4, **characterised in that** the polycarboxylic acid is an alkali-soluble polymer or copolymer of acrylic and/or methacrylic acid.

6. Ranitidine preparation according to claim 5, **characterised in that** the polycarboxylic acid is a polymer or copolymer of 10 to 100 wt.-% of units of acrylic and/or methacrylic acid and 90 to 0 wt.-% of units of one or more lower alkylesters of acrylic and/or methacrylic acid.

## Revendications

1. Préparation de ranitidine contenant le sel de ranitidine avec un acide polycarboxylique ayant un poids moléculaire (moyenne pondérale) d'au moins 1000 à 200 000 daltons.

2. Préparation de ranitidine selon la revendication 1 sous la forme d'une solution aqueuse.

3. Préparation de ranitidine selon la revendication 1 ou 2,
**caractérisée en ce que**
par mole de ranitidine elle contient au moins la quantité équivalente de groupes carboxylate de l'acide polycarboxylique.

4. Préparation de ranitidine selon la revendication 3,
**caractérisée en ce que**
l'acide polycarboxylique a un poids équivalent carboxyle de 72 à 800 daltons.

5. Préparation de ranitidine selon une ou plusieurs des revendications 1 à 4,
**caractérisée en ce que**
l'acide polycarboxylique est un polymère ou copolymères de l'acide acrylique et/ou de l'acide méthacrylique soluble dans les bases.

6. Préparation de ranitidine selon la revendication 5,
**caractérisée en ce que**
l'acide polycarboxylique est un polymère ou copolymère constitué de 10 à 100 % en poids d'unités de l'acide acrylique et/ou méthacrylique et de 90 à 0 % en poids d'unités d'un ou plusieurs esters alkyliques inférieurs de l'acide acrylique et/ou de l'acide méthacrylique.
